# EUROPEAN PATENT SPECIFICATION

(11) **EP 3 273 793 B1**
(45) Date of publication and mention of the grant of the patent: **19.08.2020**
(21) Application number: 16769804.2
(22) Date of filing: 25.03.2016
(51) Int. Cl.: A23K 10/00, A23K 20/142, A23K 20/158, A23K 50/10, A23K 40/10, A23K 40/20, A23K 40/35, A23K 20/174, A23K 20/22, A23K 20/24, A23K 20/20

(54) **AMINO ACID ANIMAL FEED COMPOSITION**
TIERFUTTERZUSAMMENSETZUNG MIT AMINOSÄURE
COMPOSITION D'ALIMENT POUR ANIMAUX À BASE D'ACIDES AMINÉS

(30) Priority: 25.03.2015 US 201562138204 P; 04.09.2015 US 201562214628 P
(43) Date of publication of application: 31.01.2018
(73) Proprietor: Benemilk Oy, 21200 Raisio (FI); Wan, Feng, Issaquah, Washington 98029 (US); Londergan, Timothy M., Seattle, WA 98105 (US)
(72) Inventor: WAN, Feng, Issaquah, WA 98029 (US); LONDERGAN, Timothy, Martin, Seattle, WA 98105 (US)
(74) Representative: Gevers Patents
(86) International application number: PCT/US2016/024317
(87) International publication number: WO 2016/154583

(56) References cited:
- WO-A1-93/10671
- WO-A1-93/10672
- WO-A1-2015/016823
- WO-A2-2004/080197
- WO-A2-2007/149818
- WO-A2-2015/016822
- CA-A1- 2 475 739
- CN-B- 101 912 034
- US-A- 5 776 483
- US-A1- 2009 252 833
- US-A1- 2010 272 852
- US-A1- 2011 200 705
- US-A1- 2012 093 974
- US-B1- 6 203 829
- US-B1- 6 203 829
- US-B2- 6 696 600
- AYLESWORTH ET AL.: 'Applications for Fatty Acids. Fatty Acids for Chemical Specialties.' A SYMPOSIUM OF THE SOAP, DETERGENTS AND SANITARY CHEMICAL PRODUCTS DIVISION OF THE CHEMICAL SPECIALTIES MANUFACTURERS ASSOCIATION. 1955, pages 142 - 146, XP009506413
- 'Palmitic acid' SIGMA-ALDRICH, [Online] 04 May 2016, pages 1 - 3, XP055487313 Retrieved from the Internet: <URL:HTTP://WWW.SIGMAALDRICH.COM/CATALOG/PR ODUCT/ALDRICH/27734?LANG=EN&REGION=US>

## Description

### BACKGROUND

Increasing production and solids content of milk obtained from lactating ruminants have been major goals for dairy farmers. Additional milk or milk solids production per ruminant is beneficial because it results in a higher yield, thereby increasing profits. Increased milk solids such as protein are desirable because it has a higher economic value and can be used in highly desirable food products, such as cheese, yogurt, and the like.

US6 203 829 B1 describes a rumen by-pass preparation for ruminants. This preparation do not include an amine-organic acid salt.

WO2004/0801197 A describes a rumen by-pass animal feed particle. This particle does not include a salt with an amine.

WO93/10671 A1 describes a process for the preparation of a dietary fatty acid salt product. This product does not include a salt with an amine.

US2012/093974 A1 describes a ruminant feed composition. This composition does not include amine-fatty acid salts.

US2010/272852 A1 also describes a ruminant feed composition which does not include amine-fatty acid salts.

CN101 912034B describes the preparation of a rumen by-pass composition. This composition does not include amine-fatty acid salts.

US2009/252833 A1 describes a control release formulation in compacted form useful in rumen by-pass dietary supplement. This formulation does not include amine-fatty acid salts.

US5 776 483 A describes a rumen by-pass formulation. This formulation does not include amine-fatty acid salts.

WO2007/149818 A2 describes a rumen by-pass dietary supplement in compacted particulate form. This supplement does not include amine-fatty acid salts.

WO93/10672 A describes a process for the preparation of a dietary fatty acid salt product. This product does not include an organic amine compound.

### SUMMARY

This summary is provided to introduce a selection of concepts in a simplified form that are further described below in the Detailed Description.

The invention relates to a rumen by-pass composition for a ruminant as defined in the appended claims.

Accordingly, the invention proposes a rumen by-pass composition for a ruminant, comprising an amine-organic acid salt including an amine compound, and
a fatty acid component, comprising an organic acid, wherein the fatty acid component has a melting point not less than 40°C and an Iodine Value not greater than 45.

As to the fatty acid component, it preferably comprises palmitic acid, stearic acid, oleic acid, linoleic acid, linolenic acid, eicosapentaenoic acid (EPA), docosahexaenoic acid (DHA), or a combination thereof.

More preferably, the fatty acid component consists essentially of palmitic acid, stearic acid, or a combination thereof.

Also preferably, the fatty acid component comprises from about 1% to about 50% by weight of an oleic acid component, the oleic component comprising oleic acid, an oleic acid ester, a high oleic oil, or a combination thereof.

As to the fatty acid component, it preferably comprises a high oleic oil, wherein the high oleic oil comprises at least 40% by weight of oleic content.

As to the organic acid, it preferably comprises palmitic acid, stearic acid, oleic acid, linoleic acid, linolenic acid, eicosapentaenoic acid (EPA), or docosahexaenoic acid (DHA), or a combination thereof.

As to the amine compound, it preferably comprises an amino acid, an amino acid derivative, or an amino acid precursor, the amino acid comprising leucine, lysine, histidine, valine, arginine, threonine, isoleucine, phenylalanine, methionine, tryptophan, carnitine, alanine, asparagine, aspartic acid, cysteine, glutamic acid, glutamine, glycine, valine, ornithine, proline, selenocysteine, selenomethionine, serine, or tyrosine.

More preferably, the amine compound comprises a lysine compound or a methionine compound, the lysine compound comprising lysine, lysine ester, lysine amide, lysine imide, or a lysine precursor, and the methionine compound comprising methionine, methionine ester, methionine amide, methionine imide, or a methionine precursor.

As to the amine-organic acid salt, it preferably comprises a salt of a lysine compound and a long chain fatty acid having a carbon chain of at least 8 carbons.

More preferably, he amine-organic acid salt comprises a salt of a methionine compound and a long chain fatty acid having a carbon chain of at least 8 carbons.

Preferably, the rumen by-pass composition of the invention, comprises particles having an outer layer encapsulating at least one inner core, the inner core comprising the amine-organic acid salt, the outer layer comprising the fatty acid component, and the inner core comprising a higher level of the amine-organic acid salt than the outer layer.

Advantageously, he rumen by-pass composition of the invention, further comprises a surfactant component, a filler, an antistatic agent, a plasticizer, a colorant, an appetite stimulant, a flavoring agent, or a combination thereof, wherein the surfactant component comprises an emulsifier having a hydrophilic-lipophilic balance value of about 5 to about 25.

Preferably, the surfactant component comprises polyoxyethylene stearate, polysorbate, polyoxyethylene sorbitan monolaurate, polyoxyethylene sorbitan monooleate, polyoxyethylene sorbitan monopalmitate, polyoxyethylene sorbitan monostearate, polyoxyethylene sorbitan tristearate, ammonium phosphatides, sodium or potassium or calcium salts of fatty acids, magnesium salts of fatty acids, mono- and diglycerides of fatty acids, acetic acid esters of mono- and diglycerides of fatty acids, lactic acid esters of mono- and diglycerides of fatty acids, citric acid esters of mono- and diglycerides of fatty acids, mono- and diacetyl tartaric acid esters of mono- and diglycerides of fatty acids, acetic acid esters of mono- and diglycerides of fatty acids, tartaric acid esters of mono- and diglycerides of fatty acids, sucrose esters of fatty acids sucroglycerides, polyglycerol esters of fatty acids polyglycerol polyricinoleate, propane-1,2-diol esters of fatty acids, thermally oxidised soya bean oil interacted with mono- and diglycerides of fatty acids, sodium stearoyl-2-lactylate, calcium stearoyl-2-lactylate, sorbitan monostearate, sorbitan tristearate, sorbitan monolaurate, sorbitan monooleate, sorbitan monopalmitate, or derivatives thereof.

More preferably, the surfactant component comprises a surfactant derived from oleic acid.

IN THIS LATTER CASE, THE SURFACTANT COMPONENT PREFERABLY COMPRISES SODIUM OLEATE, POTASSIUM OLEATE, CALCIUM OLEATE, AMMONIUM OLEATE, SORBITAN OLEATE, SORBITAN TRIOLEATE, GLYCERYL OLEATE, METHYL OLEATE, ETHYL OLEATE, PEG OLEATE, TRIETHANOLAMINE OLEATE (TEA OLEATE), POLYSORBITAN OLEATE, POLYSORBATE 20, POLYSORBATE 40, POLYSORBATE 60, POLYSORBATE 80, OR A COMBINATION THEREOF.DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1A is a diagrammatical illustration of an embodiment of a rumen by-pass composition;
FIGURE 1B is a diagrammatical illustration of an embodiment of a rumen by-pass composition;
FIGURE 2 is a schematic illustration of a method and a system, which are not part of the invention, for making a rumen by-pass composition
FIGURE 3 is a schematic illustration of a method and a system, which are not part of the invention, for making a rumen by-pass composition;
FIGURE 4 is a schematic illustration of a method and a system, which are not part of the invention, for making a rumen by-pass composition; and
FIGURE 5 is a graph showing the fractions of nitrogen remaining following three incubation phases using samples of rumen by-pass compositions.

### DETAILED DESCRIPTION

As used in this document, the singular forms "a," "an," and "the" include plural references unless the context clearly dictates otherwise. Unless defined otherwise, all technical and scientific terms used herein have the same meanings as commonly understood by one of ordinary skill in the art.

The following terms shall have, for the purposes of this application, the respective meanings set forth below.

A "ruminant" is generally a suborder of mammal with a multiple chamber stomach that gives the animal the ability to digest cellulose-based food by softening it within a first chamber (rumen) of the stomach and to regurgitate the semi-digested mass to be chewed again by the ruminant for digestion in one or more other chambers of the stomach. Examples of ruminants include, but are not limited to, lactating animals such as cattle, goats and sheep. Cattle may include dairy cows, which are generally animals of the species Bos taurus. The milk produced by ruminants is widely used in a variety of dairy-based products.

The present disclosure generally relates to rumen by-pass compositions. Herein are also described, amino acid compositions, ruminant feed mixtures, dietary compositions made therefrom, and methods for making the dietary compositions that can be fed to ruminants; but these compositions, mixtures and methods are not part of the invention. The dietary compositions may be configured to improve various aspects of milk production in the ruminants. For instance, some embodiments provide that the dietary compositions may increase the amount of milk production by the ruminant, increase the fat content of the milk produced by the ruminant, increase the protein content of the milk produced by the ruminant, or all three. Specific compositions described herein may include ruminant feed mixtures, supplements, or the like. According to some embodiments, the dietary compositions may include liquids, solids or combinations thereof, such as dry particles, pellets, liquid suspensions, emulsions, slurries, pastes, gels, or the like.

When a ruminant consumes feed, the nutrients such as fat, amino acids, and vitamins etc. in the feed may be degraded or modified by the rumen microbes. This may cause several potential undesirable effects: first, nutrients such as amino acids that are not inert in the rumen may not reach the lower digestive tract and therefore become unavailable to the ruminant; second, the nutrients such as unsaturated fat that are not inert in the rumen may have negative effect on rumen digestion and health and therefore may decrease feed intake and decrease rumen digestibility of the feed; and, third, the metabolite from the rumen metabolism of some nutrients may negatively affect milk productions.

A rumen by-pass composition, described herein, may allow for the transfer of a nutritional agent such as essential amino acids from the digestive tract into the blood circulation of a ruminant. This may remove the limiting factors in the milk production and improve energy utilization therefore increasing milk production, milk protein, milk fat, feed efficiency, or all of the above.

The disclosed rumen by-pass compositions can be configured to be protected from rumen bacterial metabolism. The composition is configured to bypass the rumen administered to the ruminant. In some embodiments, from about 40% to about 98% of the composition by-passes the rumen. In some embodiments, at least 50% of the composition by-passes the rumen. In some embodiments, at least 60% of the composition by-passes the rumen. In some embodiments, at least 70%, 80%, 90% of the composition by-passes the rumen.

In some embodiments, a rumen by-pass composition for a ruminant comprises an amine-organic acid salt including an amine compound and a fatty acid component comprising an organic acid, wherein the fatty acid component has a melting point not less than 40°C and an Iodine Value not greater than 45.

In some embodiments, the rumen by-pass composition may be in free flowing solid form. In some embodiments, the ruminant dietary composition may be a dry particle, a pellet, a liquid suspension, a paste, or an emulsion.

In some embodiments, the rumen by-pass composition may be formed as solid particles such as, without limitation, spherical beads, oval beads, flakes, granules, pellets, or a combination thereof. The particle size may have a diameter from about 1µm to about 20mm. In some embodiments, the particle size is not greater than 5mm. In some embodiments, the particle size may be from about 1µm to about 3mm, from about 1µm to about 10mm, from about 10µm to about 2mm, or from about 100µm to about 4mm. In some embodiments, the average particle size is about 1mm or about 2mm. In some embodiments, the mean particle size is about 1mm. In some embodiments, the average particle size is about 1µm to about 5mm. In some embodiments, the mean particle size is about 1µm to about 5mm.

In some embodiments, the rumen by-pass composition may have a specific density of from about 0.5 to about 2 or from about 0.8 to about 1.5. In some embodiments, the rumen by-pass composition may have a specific density of about 1. In some embodiments, the rumen by-pass composition has a specific density equal to or larger than the specific density of the rumen fluid. In some embodiments, the rumen by-pass composition has a specific density that would facilitate the rumen by-pass composition to pass through the rumen within 2, 4, 6, 8, 12, 24, 36, or 48 hours.

The rumen by-pass composition can be a homogenous mixture or a heterogeneous mixture. In some embodiments, the rumen by-pass composition is a homogeneous mixture. In some embodiments, the rumen by-pass composition is a heterogeneous mixture.

The rumen by-pass composition may have a melting point not less than 50°C, 60°C, 70°C, 80°C, 90°C, 100°C, 200°C, 300°C or 400°C. In some embodiments, the rumen by-pass composition may have a melting point from about 50°C to about 200°C, from about 55°C to about 100°C, or from about 60°C to about 90°C.

The fatty acid component may have a melting point not less than 50°C, 55°C, 60°C, 70°C, or 80°C. In some embodiments, the fatty acid component may have a melting point from about 54°C to about 200°C or from about 55°C to about 80°C. In some embodiments, the fatty acid component may have a melting point not less than 50°C. In some embodiments, the fatty acid component may have a melting point not less than 60°C.

The fatty acid component may have an Iodine Value not greater than 0.5, 1, 2, 5, 6, 7, or 10. In some embodiments, the fatty acid component may have an Iodine Value from about 0.5 to about 6. In some embodiments, the fatty acid component has an Iodine Value from about 0.5 to about 2. In some embodiments, the fatty acid component has an Iodine Value not greater than 30. In some embodiments, the fatty acid component has an Iodine Value not greater than 15. In some embodiments, the fatty acid component has an Iodine Value not greater than 5.

The fatty acid component may have a moisture level of not greater than 1%, 2%, 3% or 5% by weight. In some embodiments, the fatty acid component may have a moisture level of not greater than 0.01%.

The fatty acid component may have unsaponifiable matter no greater than 0.5%, 1%, 1.5%, 2%, 3%, 5%, or 10% by weight. In some embodiments, the fatty acid component comprises unsaponifiable matter no greater than 25% by weight. In some embodiments, the fatty acid component comprises unsaponifiable matter no greater than 15% by weight. In some embodiments, the fatty acid component comprises unsaponifiable matter no greater than 2% by weight. In some embodiments, the fatty acid component comprises unsaponifiable matter no greater than 1.5% by weight.

In some embodiments, the fatty acid component comprises a rumen stable fatty acid. The rumen stable fatty acid may be free fatty acid or esters of free fatty acid. In some embodiments, the fatty acid component may include rumen stable fatty acid not less than about 70%, 80%, 85%, 90%, 95%, 98%, or 99% by weight. In some embodiments, the fatty acid component may include free fatty acid not less than about 70%, 80%, 85%, 90%, 95%, 98%, or 99% by weight. In some embodiments, the fatty acid component comprises at least about 80% of free fatty acid by weight.

In some embodiments, the fatty acid component comprises palmitic acid, stearic acid, oleic acid, linoleic acid, linolenic acid, eicosapentaenoic acid (EPA), docosahexaenoic acid (DHA), an omega-3 fatty acid, an omega-6 fatty acid, or a combination thereof.

In some embodiments, the fatty acid component may include a palmitic acid compound. In some embodiments, the fatty acid component may include at least about 98%, 97%, 95%, 94%, 92%, 90%, 85% or 80% of a palmitic acid compound by weight. The palmitic acid compound may include free palmitic acid, a palmitic acid derivative, or both. In some embodiments, the fatty acid component includes at least 95% of free palmitic acid by weight. In some embodiments, the fatty acid component may include at least 98% of free palmitic acid by weight. The palmitic acid derivative may include a palmitic acid ester, a palmitic acid amide, a palmitic acid salt, a palmitic acid carbonate, a palmitic acid carbamates, a palmitic acid imide, a palmitic acid anhydride, or a combination thereof.

The fatty acid component may include a stearic acid compound. The stearic acid compound may include free stearic acid, a stearic acid derivative, or both. The stearic acid derivative may include a stearic acid ester, a stearic acid amide, a stearic acid salt, a stearic acid carbonate, a stearic acid carbamates, a stearic acid imide, a stearic acid anhydride, or a combination thereof.

In some embodiments, the fatty acid component may consist essentially of a palmitic acid compound, a stearic acid compound, or a combination thereof. In some embodiments, the fatty acid component may comprise a palmitic acid compound, a stearic acid compound, or a combination thereof. In some embodiments, the fatty acid component may comprise palmitic acid, stearic acid compound, or a combination thereof. In some embodiments, the fatty acid component may consist essentially of free palmitic acid and free stearic acid. In some embodiments, the fatty acid component may consist essentially of palmitic acid and stearic acid. In some embodiments, the fatty acid component may consist essentially of free palmitic acid and free stearic acid having a weight/weight ratio from about 10:1 to about 1:10. In some embodiments, the fatty acid component may comprise free palmitic acid and free stearic acid. In some embodiments, the fatty acid component may comprise palmitic acid and stearic acid. In some embodiments, the fatty acid component may comprise free palmitic acid and free stearic acid having a weight/weight ratio from about 10:1 to about 1:10. In some embodiments, the ratio of free palmitic acid and free stearic acid is about 4:6 w/w, about 7:3 w/w, about 1:1 w/w or about 9:1 w/w. In some embodiments, the ratio of free palmitic acid and free stearic acid is about 6:4 to about 4:6. In some embodiments, the ratio of free palmitic acid and free stearic acid is about 8:2 to about 2:8.

In some embodiments, the fatty acid component comprises an oleic component. In some embodiments, the oleic component comprises oleic acid, an oleic acid ester, a high oleic oil, or a combination thereof. In some embodiments, the fatty acid component comprises from about 1% to about 50% by weight of the oleic acid component. In some embodiments, the fatty acid component comprises a high oleic oil. In some embodiments, the high oleic oil comprises not less than 40% by weight of oleic content. In some embodiments, the high oleic oil comprises not less than 50% by weight of oleic content. In some embodiments, the high oleic oil comprises not less than 60% by weight of oleic content.

The organic acid may be any organic compound with acidic properties. Examples of organic acids may include carboxylic acids such as acetic acid, propionic acid, butyric acid, pentanoic acid, hexanoic acid, heptanoic acid, or octanoic acid. In some embodiments, the organic acid is a saturated fatty acid. In some embodiments, the organic acid is an unsaturated fatty acid. In some embodiments, the organic acid comprises a long chain fatty acid having a carbon chain of at least 8 carbons. In some embodiments, the organic acid comprises a long chain fatty acid having a carbon chain of at least 6 carbons. In some embodiments, the organic acid comprises a long chain fatty acid having a carbon chain of at least 6, 7, 8, 9, 10, 11, 12, 13, 14, 15, 16, 17, 18, 19, 20, or more carbons. In some embodiments, the organic acid has a pKa value of at least 2. In some embodiments, the organic acid has a pKa value of about 2, 3, 4, 4.5, 4.75, 5, 6, 7, 8, 9, 10, or 11. In some embodiments, the fatty acid has a pKa value of from about 2 to about 12 and any number in between. In some embodiments, the organic acid comprises a fatty acid having a pKa value of at least 4. In some embodiments, the organic acid comprises a fatty acid having a pKa value of from about 4 to about 11. In some embodiments, the organic acid comprises about 10%, 20%,30%, 40%, 50%, 60%, 70%, 80%, 85%, 90%, 95%, 98%, or 99% by weight of the fatty acid component.

In some embodiments, the organic acid comprises palmitic acid, stearic acid, oleic acid, linoleic acid, linolenic acid, eicosapentaenoic acid (EPA), docosahexaenoic acid (DHA), an omega-3 fatty acid, an omega-6 fatty acid, any isomer or a combination thereof. In some embodiments, the organic acid is palmitic acid. In some embodiments, the organic acid is stearic acid. In some embodiments, the organic acid is a mixture of palmitic acid and stearic acid.

In some embodiments, the amine compound comprises a compound having a primary, secondary, or tertiary amine group. In some embodiments, the amine group is configured to form a salt with a fatty acid having a pKa value of at least 4. The amine group may be strong enough or in unbound state such that the amine group is configured to form the amine-organic acid salt with a fatty acid. For example, because fatty acids tend to have a weaker pKa value than inorganic acids, in order for the amine group to form a salt with a fatty acid, the amine group may not be bound in a salt form with a strong acid. In some embodiments, the strong acid comprises an acid having a pKa value of less than about 0, 1, 2, or 3. Example strong acids may include without limitation HC1, HBr, HI, H₂SO₄, HNO₃, and H₃PO₄.

In some embodiments, the amine compound comprises an amino acid compound. In some embodiments, the amine compound comprises an amino acid, an amino acid derivative, or an amino acid precursor. In some embodiments, the amino acid comprises leucine, lysine, histidine, valine, arginine, threonine, isoleucine, phenylalanine, methionine, tryptophan, carnitine, alanine, asparagine, lysine, aspartic acid, cysteine, glutamic acid, glutamine, glycine, valine, ornithine, proline, selenocysteine, selenomethionine, serine, or tyrosine, or any derivatives from the foregoing list. The amino acid may be any essential or non essential amino. The amino acid may be metal chelated amino acids. In some embodiments, the amino acid may be an amino acid chelated or glycinated with mineral or selenium yeast. For example, the amino acid may be chelated with Zn, Fe , Ca, Se or Cobalt.

In some embodiments, the amine compound comprises a lysine compound or a methionine compound. In some embodiments, the lysine compound comprises lysine, lysine ester, lysine amide, lysine imide, or a lysine precursor. In some embodiments, the methionine compound comprises methionine, methionine ester, methionine amide, methionine imide, or a methionine precursor.

In some embodiments, the amine-organic acid salt comprises a salt of a lysine compound and a long chain fatty acid having a carbon chain of at least 8 carbons. In some embodiments, the long chain fatty acid has more than 8 carbons, including 9, 10, 11, 12, 13, 14, 15, 16, 17, 18, 29, 20, or more carbons. In some embodiments, the amine-organic acid salt comprises a salt of a lysine compound and a fatty acid having a pKa value of at least 4. A lysine compound may be selected from a lysine, an ester, an amide, an imide, a metal chelated lysine derivative, or a combination thereof. The metal chelated lysine derivative comprises a lysine chelated with a metal selected from calcium, sodium, magnesium, phosphorous, potassium, manganese, zinc, selenium, copper, iodine, iron, cobalt, or molybdenum, or a combination thereof. In some embodiments, the amine-organic acid salt comprises a salt of a lysine compound and palmitic acid. In some embodiments, the amine-organic acid salt comprises a salt of a lysine compound and stearic acid. In some embodiments, the amine-organic acid salt comprises a lysine-palmitic acid salt. In some embodiments, the amine-organic acid salt comprises a lysine-stearic acid salt.

In some embodiments, the amine-organic acid salt comprises a salt of a methionine compound and a long chain fatty acid having a carbon chain of at least 8 carbons. In some embodiments, the long chain fatty acid has more than 8 carbons, including 9, 10, 11, 12, 13, 14, 15, 16, 17, 18, 29, 20, or more carbons. In some embodiments, the amine-organic acid salt comprises a salt of methionine compound and a fatty acid having a pKa value of at least 4. A methionine compound may be selected from an ester, a thioester, a disulfide derivative, an ether, a thioether, an amide, an imide, a metal chelated methionine derivative, or a combination thereof. The metal chelated methionine derivative may include a methionine chelated with a metal selected from calcium, sodium, magnesium, phosphorous, potassium, manganese, zinc, selenium, copper, iodine, iron, cobalt, or molybdenum, or a combination thereof. In some embodiments, the amine-organic acid salt comprises a salt of a methionine compound and palmitic acid. In some embodiments, the amine-organic acid salt comprises a salt of a methionine compound and stearic acid. In some embodiments, the methionine compound comprises methionine methyl ester or methionine ethyl ester.

In some embodiments, the rumen by-pass compositions are particles comprising at least one inner core and an outer layer. In some embodiments, the outer layer encapsulates at least one inner core, wherein the inner core comprises the amine-organic acid salt. In some embodiments, the outer layer comprises the fatty acid component. In some embodiments, the inner core comprises a higher level of the amine-organic acid salt than the outer layer. In some embodiments, the outer layer comprises a wax. In some embodiments, the outer layer comprises a polymer. In some embodiments, there are two or more layers surrounding an inner core.

In some embodiments, the wax may include without limitation a paraffin wax, a natural wax, a synthetic wax, a microcrystalline wax, or a combination thereof. The natural wax may comprise without limitation carnauba wax, beeswax, petroleum wax, rice bran wax, castor wax, their derivatives, or a combination thereof.

In some embodiments, the polymer may comprise a cross-linked polymer. In some embodiments, the polymer may include without limitation polyurethane, polyester, polystyrene, polypyridine, polyvinylpyridine, polycyanate, polyisocynate, polysaccharide, polynucleotide, polyethylene, polyisobutylene, polyvinyl acetate, protein, polysaccharide, or a combination thereof.

In some embodiments, the polymer may comprise a denatured protein. In some embodiments, the polymer may comprise a cross-linked protein. In some embodiments, the protein may be cross-linked by reducing sugars. Representative reducing sugars may include without limitation glucose, lactose, fructose, mannose, maltose, ribose, galactose, their derivatives, or a combination thereof. In some embodiments, the protein may be cross-linked by heat-induced formation of disulfide bonds. In some embodiments, the protein may be cross-linked by disulfide bonds, hydrophobic interactions, ionic interactions, hydrogen bonding, or a combination thereof. In some embodiments, the protein may be cross-linked with a divalent linker, formaldehyde, glutaraldehyde, or other aldehydes.

The cross-linked polymer may comprise a vegetable oil. The vegetable oil may be a cross-linked vegetable oil. In some embodiments, the outer layer of particles may comprise a cross-linked vegetable oil. In some embodiment, the cross-linked vegetable oil may be cross-linked through a divalent linker. In some embodiments, the cross-linked vegetable oil may comprise cross-linked corn oil, cross-linked cottonseed oil, cross-linked peanut oil, cross-linked palm kernel oil, cross-linked soybean oil, cross-linked rapeseed oil, cross-linked sunflower oil, or a combination thereof.

The rumen by-pass composition may further comprise a surfactant component, a filler, an antistatic agent, a plasticizer, a colorant, an appetite stimulant, a flavoring agent, or a combination thereof.

In some embodiments, the rumen by-pass composition comprises not more than about 30% of the surfactant component. In some embodiments, the rumen by-pass composition comprises no more than about 10% of the surfactant component. In some embodiments, the rumen by-pass composition comprises no more than about 5% of the surfactant component.

In some embodiments, the surfactant component may be a nonionic surfactant or an ionic surfactant. In some embodiments, the surfactant component comprises a non-ionic emulsifier. In some embodiments, the surfactant component comprises an ionic emulsifier. In some embodiments, the surfactant component may comprise an emulsifier having a hydrophilic-lipophilic balance value of about 2 to about 12, about 5 to about 14, about 2 to about 8, or about 6 to about 14. In some embodiments, the surfactant component may comprise an emulsifier having a hydrophilic-lipophilic balance value of not greater than about 10. In some embodiments, the surfactant component comprises an emulsifier having a hydrophilic-lipophilic balance value of about 5 to about 25. In some embodiments, the surfactant component comprises an emulsifier having a hydrophilic-lipophilic balance value of from about 10 to about 20. In some embodiments, the surfactant component comprises an emulsifier having a hydrophilic-lipophilic balance value of at least about 7. In some embodiments, the surfactant component comprises an emulsifier having a hydrophilic-lipophilic balance value of about 15. In some embodiments, the surfactant component may include lecithin, soy lecithin, cephalin, castor oil ethoxylate, sorbitan mono-, di-, or trioleate, polysorbitan mono-, di- or trioleate, tallow ethoxylate, lauric acid, polyethylene glycol, or derivatives thereof.

In some embodiments, the surfactant component may include calcium stearoyl dilaciate, glycerol ester, polyglycerol ester, sorbitan ester, polysorbitan ester, polyethylene glycol ester, sugar ester, mono-, di-, or triglyceride, acetylated monoglyceride, lactylated monoglyceride, or derivatives thereof.

In some embodiments, the surfactant component may include castor oil, lecithin, polysorbate, an ammonia solution, butoxyethanol, propylene glycol, ethylene glycol, ethylene glycol polymers, polyethylene, methoxypolyethylene glycol, soy lecithin, cephalin, castor oil ethoxylate, sorbitan monooleate, tallow ethoxylate, lauric acid, polyethylene glycol, calcium stearoyl dilaciate, polyglycerol ester, sorbitan ester, polyethylene glycol ester, sugar ester, monoglyceride, acetylated monoglyceride, lactylated monoglyceride.

In some embodiments, the surfactant component comprises polyoxyethylene stearate, polysorbate, polyoxyethylene sorbitan monolaurate, polyoxyethylene sorbitan monooleate, polyoxyethylene sorbitan monopalmitate, polyoxyethylene sorbitan monostearate, polyoxyethylene sorbitan tristearate, ammonium phosphatides, sodium or potassium or calcium salts of fatty acids, magnesium salts of fatty acids, mono- and diglycerides of fatty acids, acetic acid esters of mono- and diglycerides of fatty acids, lactic acid esters of mono- and diglycerides of fatty acids, citric acid esters of mono- and diglycerides of fatty acids, mono- and diacetyl tartaric acid esters of mono- and diglycerides of fatty acids, acetic acid esters of mono- and diglycerides of fatty acids, tartaric acid esters of mono- and diglycerides of fatty acids, sucrose esters of fatty acids sucroglycerides, polyglycerol esters of fatty acids polyglycerol polyricinoleate, propane-1,2-diol esters of fatty acids, thermally oxidised soya bean oil interacted with mono- and diglycerides of fatty acids, sodium stearoyl-2-lactylate, calcium stearoyl-2-lactylate, sorbitan monostearate, sorbitan tristearate, sorbitan monolaurate, sorbitan monooleate, sorbitan monopalmitate, or derivatives thereof.

In some embodiments, the sodium or potassium or calcium salts of fatty acids comprises sodium or potassium or calcium salts of distilled palm fatty acids.

In some embodiments, the surfactant component comprises a surfactant derived from oleic acid. In some embodiments, the surfactant component comprises a non-ionic oleate ester derived surfactant. In some embodiments, the surfactant component comprises an ionic oleic acid derived surfactant. In some embodiments, the surfactant component comprises sodium oleate, potassium oleate, calcium oleate, ammonium oleate, sorbitan oleate, sorbitan trioleate, glyceryl oleate, methyl oleate, ethyl oleate, PEG oleate, triethanolamine oleate (TEA oleate), polysorbitan oleate, polysorbate 80, or a combination thereof.

In some embodiments, the surfactant component may include Tween 20 (polysorbate laureate), Tween 40 (polysorbate 40 palmitate), Tween 60 (polysorbate stearate), Tween 80 (polysorbate oleate), or a combination thereof. In some embodiments, the surfactant component may include a bredol surfactant. In some embodiments, the surfactant component may be a liquid surfactant. In some embodiments, the surfactant component may be a solid surfactant.

The surfactant component may be present in the rumen by-pass composition in an amount of about 0.01% by weight to about 50.0% by weight. In some embodiments, the weight/weight ratio of the surfactant component to the fatty acid component may be about 1:1000 to about 1:5.

In some embodiments, the rumen by-pass composition can have a weight/weight ratio of the surfactant component to the fatty acid component of about 1:50 to about 1:20, about 1:100 to about 1:5, about 1:1000 to about 1:2, or about 1:1 to about 1:100. In some embodiments, the ratio may be about 1:10.

The rumen by-pass composition may include no more than 2%, 5%, 15%, 25%, or 30% by weight of the surfactant component. In some embodiments, the rumen by-pass composition comprises from about 0.01% to about 25% by weight of the surfactant component.

The filler may include a feed material, or a mineral. Representative feed materials may include without limitation grain, roughage, forage, silage, a protein material, a carbohydrate material, or a combination thereof. In some embodiments, the feed material may include wheat, grains, rapeseed meal, soybean meal, sunflower meal, cottonseed meal, camelina meal, mustard seed meal, crambe seed meal, safflower meal, rice meal, peanut meal, corn gluten meal, corn gluten feed, distillers dried grains, distillers dried grains with solubles, wheat gluten, wheat bran, wheat middlings, wheat mill run, wheat mill run, oat hulls, soya hulls, grass meal, hay meal, alfalfa meal, alfalfa, straw, hay, or a combination thereof.

The protein material may include rapeseed meal, soybean meal, sunflower meal, cottonseed meal, camelina meal, mustard seed meal, crambe seed meal, safflower meal, rice meal, peanut meal, corn gluten meal, corn gluten feed, wheat gluten, distillers dried grains, distillers dried grains with solubles, animal protein, or a combination thereof. In some embodiments, the protein material may include blood meal, crab protein concentrate, fish meal, hydrolyzed poultry feather meal, soybean meal, soybean protein concentrate, sunflower seed meal, cotton seed meal, corn gluten meal, alfalfa residues, brewer's residues, meat and bone meal, meat meal, canola meal and poultry by-product meal, or a combination thereof. In some embodiments, the protein material comprises soybean meals, rapeseed meals, sunflower meals, coconut meals, olive meals, linseed meals, grapeseed meals, cottonseed meals, or mixtures thereof. In some embodiments, the protein material may include denatured protein. In some embodiments, the protein material may include cross-linked protein. In some embodiments, the protein material may include partially hydrolyzed protein.

In some embodiments, the antistatic agent may be a salt, oil, or a mineral.

In some embodiments, the plasticizer includes a dispersant. In some embodiments, the plasticizer comprises starch, silicon dioxide, hydrophilic silica, or a combination thereof.

The colorant may be a food or feed grade dye, an antioxidant, a vitamin, a mineral, or a combination thereof. In some embodiments, the colorant comprises an antioxidant. In some embodiments, the colorant comprises a flavone, a quinone, a flavanone, an anthracene, a plant extract, a fruit extract, a vitamin, or a combination thereof.

In some embodiments, the appetite stimulant comprises Vitamin B, Vitamin B6, Vitamin B12, molasses, probiotics, prebiotics, nutritional yeast, or a combination thereof.

In some embodiments, the flavoring agent comprises an aliphatic alcohol, an aromatic alcohol, an ether, a furan ether, a thiazole alcohol, a pyridine ether, a pyridine alcohol, a benzofuran carbonyl compound, an aliphatic ketone, an aromatic ketone, a α-diketone, a pyrrole-α-diketone, an aromatic sulfur compound, a phenol, an phenol ether, an essential oil, or a derivative thereof. In some embodiments, the flavoring agent comprises bubble gum flavor, butter scotch flavor, cinnamon flavor, or a combination thereof. In some embodiments, the flavoring agent comprises anethole, benzaldehyde, bergamot oil, acetoin, carvol, cinnamaldehyde, citral, ethylvanillin, vanillin, thymol, methyl salicylate, coumarin, anise, cinnamon, ginger, clove, lemon oil, 1-undecanol, 5-dodecalactone, eugenol, geraniol, geranyl acetate, guaiacol, limonene, linalool, piperonal, 2-acetyl-5-methylpyrazine, 2-ethyl-3-methoxypyrazine, 5-methylquinoxaline, 2methyl-6-propylpyrazine, 2-methylbenzofuran, 2,2'-dithienylmethane, benzyl hexyl carbinol, furfuryl phenyl ether, difurfuryl ether, benzofuran-2-aldehyde, benzothiophene-2-aldehyde, 1-butylpyrrole-2-aldehyde, methyl decyl ketone, dipropyl ketone, ethyl benzyl ketone, 2,6-diacetylpyridine, heptane-3,4-dione, methyl thiophene-2-carboxylate, 2-hydroxyacetophenone, 4-ethyl-2-methoxyphenol, 2-oxobutan-1-ol, or a derivative thereof.

In another aspect, a dietary composition for ruminants includes any one of the rumen by-pass compositions and a feed material. In some embodiments, the dietary composition is in the form of a mash mixture, particles, granules, flakes, or pellets.

In some embodiments, the dietary composition has a moisture level of not greater than 30%. In some embodiments, the dietary composition has a moisture level of not greater than 20%. In some embodiments, the dietary composition has a moisture level of not greater than 12%.

In some embodiments, the feed material comprises a nutritional agent, a roughage, a forage, a silage, a grain, or an oilseed meal.

In some embodiments, the nutritional agent comprises an antioxidant, a bioactive agent, a flavoring agent, a colorant, a glucogenic precursor, a vitamin, a mineral, an amino acid, a trace element, a lipid, a prebiotic agent, a probiotic agent, an antimicrobial agent, an enzyme, a choline derivative, or derivatives thereof.

In some embodiments, the antioxidant comprises ethoxyquin (1,2-dihydro-6-ethoxy-2,2,4-trimethylquinoline), BHA (butylated hydroxyanisole), BHT (butylated hydroxytoluene), ascorbic acid, ascorbyl palmitate, benzoic acid, calcium ascorbate, calcium propionate, calcium sorbate, citrate acid, dilauryl thiodipropionate, distearyl thiodipropionate, erythorbic acid, formic acid, methylparaben, potassium bisulphite, potassium metabisulphite, potassium sorbate, propionic acid, propyl gallate, propyl paraben, resin guaiae, sodium ascorbate, sodium benzoate, sodium bisulphite, sodium metabisulphite, sodium nitrite, sodium propionate, sodium sorbate, sodium sulphite, sorbic acid, stannous chloride, sulphur dioxide, THBP (trihydroxy-butyrophenone), TBHQ (tertiary-butylhydroquinone), thiodipinic acid, tocopherols, polyphenol, carotenoid, flavonoids, flavones, quinones, anthracenes, or derivatives thereof.

In some embodiments, the bioactive agent comprises a prebiotic agent, a probiotic agent, or an antimicrobial agent.

In some embodiments, the prebiotic agent comprises fructo-oligosaccahrides, inulin, galacto-oligosaccahride, mannan-oligosaccahride, a yeast or yeast derivative, a component of a yeast, a yeast extract, or a combination thereof.

In some embodiments, the probiotic agent comprises lactic acid-producing bacteria, live yeast cells, yeast culture, enzymes, protease, amylase, or a combination thereof.

In some embodiments, the antimicrobial agent comprises monensin, bambermycin, lasalocid, salinomycin, a sesquiterpene, a terpene, an alkaloid, an essential oil, or their derivative thereof.

In some embodiments, the glucogenic precursor comprises glycerol, propylene glycol, molasses, vinasses, glycerine, propanediol, polyol, or calcium or sodium propionate.

In some embodiments, the vitamin comprises biotin, vitamin A, vitamin C, vitamin D, vitamin E, vitamin H, vitamin K, vitamin B₁, vitamin B₂, vitamin B₃, vitamin B₅, vitamin B₆, vitamin B₇, vitamin B₉, vitamin B₁₂, or vitamin Bₚ. In some embodiments, the vitamin may include thiamine, riboflavin, niacin, pantothenic acid, pyridoxine, biotin, folic acid, cobalamin, carnitine, choline, or its derivative thereof.

In some embodiments, the mineral comprises a salt of calcium, sodium, magnesium, potassium, phosphorus, zinc, selenium, manganese, iron, cobalt, copper, iodine, molybdenum, an amino acid chelated mineral, an amino acid glycinated mineral, selenium yeast, an organic mineral chelate, an organic mineral glycinate, or a combination thereof.

In some embodiments, the mineral comprises an amino acid chelated or glycinated mineral or selenium yeast.

In some embodiments, the mineral comprises an organic mineral derivative.

In some embodiments, the mineral comprises copper sulfate, copper oxide, and/or a chelated mineral.

The mineral may include any organic or inorganic salt. Representative minerals include a salt of calcium, sodium, magnesium, potassium, phosphorus, zinc, selenium, manganese, iron, cobalt, copper, iodine, molybdenum, an amino acid chelated mineral, an amino acid glycinated mineral, selenium yeast, an organic mineral chelate, an organic mineral glycinate, or a combination thereof. In some embodiments, the mineral is an organic mineral derivative. In some embodiments, the mineral comprises a sodium salt selected from monosodium phosphate, sodium acetate, sodium chloride, sodium bicarbonate, disodium phosphate, sodium iodate, sodium iodide, sodium tripolyphosphate, sodium sulfate, and sodium selenite. In some embodiments, the mineral comprises a calcium salt selected from calcium acetate, calcium carbonate, calcium chloride, calcium gluconate, calcium hydroxide, calcium iodate, calcium iodobehenate, calcium oxide, anhydrous calcium sulfate, calcium sulfate dehydrate, dicalcium phosphate, monocalcium phosphate, and tricalcium phosphate. In some embodiments, the mineral comprises a magnesium salt selected from magnesium acetate, magnesium carbonate, magnesium oxide, and magnesium sulfate. In some embodiments, the mineral comprises a cobalt salt selected from cobalt acetate, cobalt carbonate, cobalt chloride, cobalt oxide, and cobalt sulfate. In some embodiments, the mineral comprises a manganese salt selected from manganese carbonate, manganese chloride, manganese citrate, manganese gluconate, manganese orthophosphate, manganese oxide, manganese phosphate, and manganese sulfate. In some embodiments, the mineral comprises a potassium salt selected from potassium acetate, potassium bicarbonate, potassium carbonate, potassium chloride, potassium iodate, potassium iodide, and potassium sulfate. In some embodiments, the mineral comprises an iron salt selected from iron ammonium citrate, iron carbonate, iron chloride, iron gluconate, iron oxide, iron phosphate, iron pyrophosphate, iron sulfate, and reduced iron. In some embodiments, the mineral comprises a zinc salt selected from zinc acetate, zinc carbonate, zinc chloride, zinc oxide, and zinc sulfate. In some embodiments, the mineral comprises copper sulfate, copper oxide, selenium yeast, and a chelated mineral.

In some embodiments, the choline derivative comprises choline, choline chloride, choline bi-tartrate, di-hydrogenated citrate of choline, bicarbonate of choline, choline sulphate, choline hydroxide, or a combination thereof.

The lipid may include one or more oils, fats, monoglycerides, diglycerides, triglycerides, or organic acids. In some embodiments, the lipid can comprise from about 5% to about 50% conjugated linoleic acid. In some embodiments, the lipid can comprise at least 25% conjugated linoleic acid. The conjugated linoleic acid compound may include any conjugated linoleic acid isomers. Example conjugated linoleic acid isomers may include trans-10, cis-12 conjugated linoleic acid, cis-8, trans-10 conjugated linoleic acid, trans-8, cis-10 conjugated linoleic acid, a conjugated linoleic acid compound comprising a double bond including carbon number 10, or a mixture comprising at least two of the above compounds.

In some embodiments, the lipid may include corn oil, poppy seed oil, fish oil, cotton seed oil, soybean oil, walnut oil, safflower oil, sunflower oil, sesame oil, canola oil, linseed oil or a combination thereof. In some embodiments, the lipid may include a vegetable oil selected from the group consisting of vegetable oils containing at least 50% C18:2 and at least 30% C18:3. In some embodiments, the lipid may include fatty acids selected from the group consisting of oleic acid, conjugated linoleic acid, linolenic acid, phytanic acid, omega 3 fatty acids, docosahexaenoic acid, eicosapentaenoic acid, their derivatives, or a combination thereof.

In some embodiments, the lipid may include one or more oils, fats, monoglycerides, diglycerides, triglycerides, organic acids, oleic acid, conjugated linoleic acid, linolenic acid, phytanic acid, omega 3 fatty acids, C22:6 fatty acids, eicospentaenoic acid (C20:5), corn oil, poppy seed oil, fish oil, cotton seed oil, peanut oil, palm oil, marine lipids, soybean oil, walnut oil, safflower oil, sunflower oil, sesame oil, canola oil or linseed oil.

In some embodiments, the dietary composition may be a premix. In some embodiments of the dietary composition, the amine-organic acid salt is derived from an amine compound and an organic acid, wherein the organic acid comprise a long chain fatty acid having a carbon chain of at least 8 carbons. In some embodiments, of the dietary composition, the amine-organic acid salt is derived from an amine compound and an organic acid, wherein the organic acid comprise a fatty acid having a pKa value of at least 4.

Total mixed ration feeding is generally understood to be the practice of combining all the ingredients the cow (or other ruminant) needs into a complete feed. For example, each bite or particle of feed includes all the grains, proteins, vitamins, minerals, etc. to meet the dietary needs of the cow (or other ruminant). This way, the cow (or other ruminant) cannot pick and choose what it will eat.

In some embodiments, the amine-organic acid salt is derived from an amine compound and an organic acid. In some embodiments, the organic acid comprises a long chain fatty acid having a carbon chain of at least 8 carbons. In some embodiments, the organic acid comprises a long chain fatty acid having a carbon chain of at least 9, 10, 11, 12, 13, 14, 15, 16, 17, 18, 19, 20 or more carbons. In some embodiments, the organic acid may have a pKa value of at least 4.

In another aspect, the application discloses methods, which are not part of the invention, for making the rumen by-pass compositions. The methods may include without limitation spray mixing, mixing with heating, coating, spraying coating, spin coating, prilling, encapsulation, or a combination thereof.

In another aspect, the application discloses systems, which are not part of the invention, for making the ruminant feed. The system may include a prilling tower, airdrying apparatus, spray coating apparatus, or a combination thereof.

In a further aspect, the application discloses methods, which are not part of the invention, of increasing milk solids content of milk produced by a ruminant. In some embodiments, the method includes the steps of providing a rumen by-pass composition to the ruminant for ingestion; and collecting milk from the ruminant after the ruminant has ingested the ruminant feed mixture, wherein milk collected from the ruminant has a higher milk fat content, milk fat yield, milk protein content, or milk protein yield compared to milk before the ruminant ingested the ruminant feed mixture.

The ruminant may be a cow, goat, or sheep.

The application further discloses methods, which are not part of the invention, for altering the concentration of milk solids in milk produced by a lactating mammal.

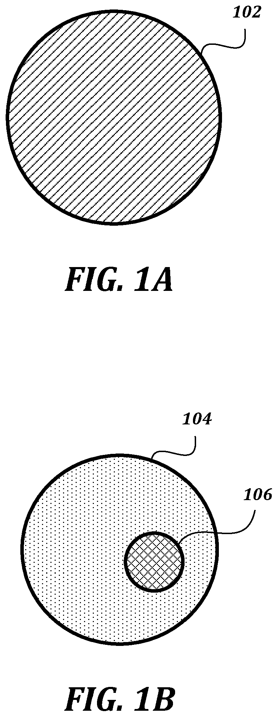

FIGURE 1A is a diagrammatical illustration of homogeneous rumen by-pass composition 102 showing uniform distribution of the amine compound and the fatty acid component throughout the rumen by-pass composition. The illustrated shape of the rumen by-pass composition is not limiting and can take other shapes depending, for example, on the manufacturing method.

FIGURE 1B is a diagrammatical illustration of heterogeneous rumen by-pass composition showing a non-uniform distribution of the amino acid compound and the fatty acid component. In the embodiment illustrated, the amine compound is shown as a core 106 and the fatty acid component is shown as the outer layer 104. In other embodiments, there is a amine-organic acid salt at the core 106 and the fatty acid component is shown as the outer layer 104. In some embodiments, the inner core 106 comprises a higher level of the amine-organic acid salt than the outer layer 104. The rumen by-pass composition of FIGURE 1B can be made, for example, by encapsulation methods. The encapsulation process may result in partial or whole encapsulation of the core. The illustrated shape of the rumen by-pass composition is not limiting and can take other shapes depending, for example, on the manufacturing method.

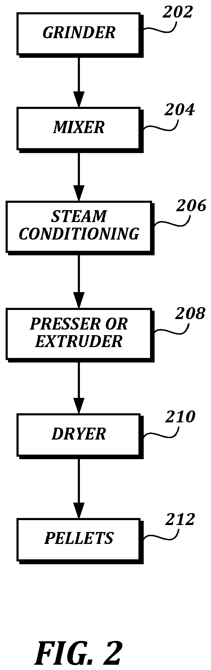

FIGURE 2 is a schematic illustration of a system and method, , which are not part of the invention, of making some embodiments of the rumen by-pass compositions and the dietary compositions. The system of FIGURE 2 may be used to make the homogeneous rumen by-pass compositions or the cores of the rumen by-pass compositions, for example. In some embodiments, the system may be used to combine the amine compound with the fatty acid component. In some embodiments, the system can include a grinder, block 202. Following the grinder, block 202, the system may include a mixer, block 204. The mixer, block 204, can include a paddle mixer or a ribbon mixer. In some embodiments, the system includes a steam conditioning vessel, block 206, in communication with the first mixer, block 204. In some embodiments, following the steam conditioning vessel, the system can include a pellet presser, expander, or extruder, block 208, in communication with the steam conditioning vessel, block 206. In some embodiments, a dryer, block 210 follows the pellet presser, expander, or extruder. The method and system of FIGURE 2 can create pellet-shaped rumen by-pass compositions.

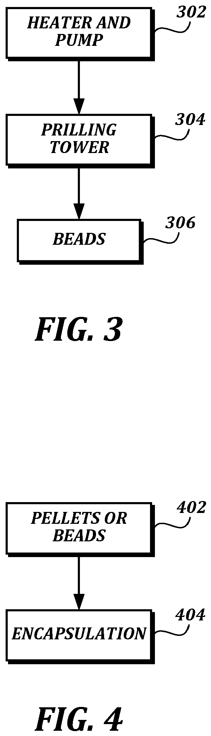

FIGURE 3 is a schematic illustration of a system and method, which are not part of the invention, of making embodiments of the rumen by-pass compositions and the dietary compositions. In some embodiments, the system may be used to combine the amine compound with the fatty acid component. One embodiment of the method employed for making the compositions is referred to as "prilling." Prilling, also called "spray chilling," "spray cooling," or "spray congealing," generally refers to a process of spraying droplets through nozzles and allowing droplets to congeal in mid-air as they fall from the top of a prilling tower toward a collection surface. Air may be circulated upward through the tower to aid in congealing the droplets into a solid. The size and shape of the droplets may be affected by the height of the tower, the nozzle size, and the nozzle shape. For example, larger sized droplets may require a higher tower than smaller sized droplets. The droplets tend to congeal without agglomerating, and the surface tension of the liquid droplets results in a generally rounded bead surface. In some embodiments, the beads may be round or oval shaped. The system of FIGURE 3 may be used to make the homogeneous rumen by-pass compositions or the cores of the rumen by-pass compositions or make encapsulated rumen by-pass compositions, for example.

In prilling, the material is heated to the melting temperature using a heater, block 302. The temperature leaving the heater can be at or slightly above the melt temperature. The melt can be pumped via a pump, block 302. Then, the melt is distributed through a droplet-producing device at the top of the prilling tower, block 304. As the droplets fall in the tower, the droplets will congeal and solidify by the time they reach the bottom of the tower as solid beads, block 306. Prilling may also be used to make encapsulated compositions. For example, during the descent of the droplets, the encapsulation layer can be sprayed with the encapsulating material from a different nozzle placed at a lower elevation. Multiple encapsulation layers may be produced by spraying different materials at different elevations.

FIGURE 4 is a schematic illustration of a system and method, which are not part of the invention, of encapsulation. In some embodiments, the beads or pellets, block 402, produced by the methods of FIGURES 2 and 3 may be further encapsulated with an encapsulation process, block 404. In some embodiments, the encapsulated rumen by-pass compositions may be manufactured with an encapsulation prilling process, block 404, in which the core material and the outer layer material are sprayed from different nozzles. In some embodiments, the rumen by-pass composition may be encapsulated with a curtain process. Other example encapsulation processes, block 404, may include, without limitation, extrusion, co-extrusion, pan coating, fluidized bed, and coacervation.

### EXAMPLES

### EXAMPLE 1: PREPARATION OF PALMITIC ACID/L-METHIONINE COMPOSITIONS

### i. Palmitic acid /L-methionine ethyl ester composition (molar ratio=1: 1)

Completely dissolve palmitic acid in a solvent with heat as needed. Completely dissolve L-methionine ethyl ester solid in a solvent. Slowly drip the methionine ethyl ester solution into palmitic acid solution while stirring. The reaction mixture was concentrated under reduced pressure to remove the solvents to provide of L-methionine ethyl ester/PA salt. Label the sample as 031A.

### ii. Palmitic acid /L-methionine ethyl ester composition (molar ratio=1:1)

Stir together the melted Palmitic acid and L-methionine ethyl ester to make palmitic acid-methionine ethyl ester salt. Cool the sample into a solid. Grind the solid into powder. Label the sample as 031B.

### iii. Palmitic acid/L-methionine ethyl ester HC1 composition (molar ratio=1: 1)

Completely dissolve palmitic acid in an organic solvent with heat as needed. Place L-methionine ethyl ester HC1 solid (0.5 mol) into a flask. Slowly drip the palmitic acid solution onto L-methionine ethyl ester HCl solid while stirring and under vacuum. The vacuum removed the solvent causing the palmitic acid to be coated on L-methionine ethyl ester particles. Label the sample as 031C.

### iv. Palmitic acid/L-methionine ethyl ester HCl composition (molar ratio=1:1)

Stir together the melted Palmitic acid and L-methionine ethyl ester HCl. Cool the sample into a solid. Label the sample as 031D.

### v. Palmitic acid/L-methionine composition (molar ratio=1: 1)

A clear solution of L-methionine in water was added to a solution of Palmitic acid in isopropyl alcohol with vigorous stirring. Stirring continued for 12 hrs. The reaction mixture was concentrated under reduced pressure to remove the solvents. The dry solid was crushed to make a fine powder. Label the sample as 031E.

### EXAMPLE 2: PREPARATION OF PALMITIC ACID, STEARIC ACID AND L-METHIONINE COMPOSITIONS

### i. PALMAC 640 /L-methionine composition (molar ratio=1:1)

Completely dissolve PALMAC 640 (a mixture of 60% palmitic acid and 40% stearic acid, 0.5 mol) in an organic solvent with heat as needed. Place L-methionine solid (0.5 mol) into a flask. Slowly drip the PALMAC 640 solution onto L-methionine solid while stirring and under a flow of air or under vacuum. The vacuum removed the solvent causing PALMAC 640 to be coated on L-methionine particles. Label the sample as 338A.

### ii. PALMAC 640/L-methionine composition (molar ration=2:1)

Completely dissolve PALMAC 640 (1 mol) in an organic solvent with heat as needed. Place L-methionine solid (0.5mol) into a flask. Slowly drip the PALMAC 640 solution onto L-methionine solid while stirring and under vacuum. The vacuum removed the solvent causing PALMAC 640 to be coated on L-methionine particles. Label the sample as 338B.

### EXAMPLE 3: PREPARATION OF PALMITIC ACID/METHIONINE COMPOSITIONS

### i. Palmitic acid /L-methionine composition (molar ratio=1:1)

A solution of palmitic acid (PALMAC 98-16) 128.2 grams (0.5 mole) in isopropyl alcohol (650 ml) was added to methionine solid (74.6 g; 0.5 mole) in a one liter flask. The flask was rotated by placing in a rotary evaporator so that the solids were uniformly coated with isopropyl solution. After stirring for 10 minutes, the solvent was removed at reduced pressure at 40-45°C. The resulting solid was crushed to a powder. Yield = 201 grams. Label the sample as 758A.

### ii. Palmitic acid /L-methionine composition (molar ration=2:1)

A solution of palmitic acid (PALMAC 98-16) 256.42 grams (1.0 mole) in isopropyl alcohol (1300 ml) was added to methionine solid (74.6 g; 0.5 mole) in a one-liter flask. The flask was rotated by placing in a rotary evaporator so that the solids were uniformly coated with isopropyl solution. After stirring for 10 minutes, the solvent was removed at reduced pressure at 40-45°C. The resulting solid was crushed to a powder. Yield = 329 grams. Label the sample as Benemilk 758B.

### EXAMPLE 4: LYSINE AND PALMITIC ACID COMPOSITIONS

### i. Preparation of palmitic acid / L-Lysine composition (molar ratio=1: 1)

A clear solution of Lysine (146 grams; 1mole) in water (250 ml) was added to a solution of palmitic acid (256 grams; 1 mol) in 1.4 liters of isopropyl alcohol with vigorous stirring. A white thick precipitate was formed immediately on addition with a slightly exothermic reaction. Stirring continued for 12 hrs. The reaction mixture was concentrated under reduced pressure to remove the solvents. The dry solid was crushed to make a fine powder of L-lysing/palmitic acid salt. Yield 383 grams. Label the sample 150A.

### ii. Preparation of palmitic acid L-Lysine composition (molar ration=2:1)

A clear solution of Lysine (146 grams; 1mole) in water (250 ml) was added to a solution of palmitic acid (512 grams; 2 mol) in 3 liters of isopropyl alcohol with vigorous stirring. A white thick precipitate was formed immediately on addition with a slightly exothermic reaction. Stirring continued for 12 hrs. The reaction mixture was concentrated under reduced pressure to remove the solvents. The dry solid were crushed to make a fine powder of palmitic acid encapsulated L-lysing/palmitic acid salt. Yield 638 grams. Label the sample 150B.

### EXAMPLE 5: LYSINE, PALMITIC ACID AND STEARIC ACID COMPOSITIONS

### i. PALMAC 640 /L-Lysine composition (molar ratio=1: 1)

A solution of PALMAC 640 (a mixture of 60% palmitic acid and 40% of stearic acid) 137.72 grams (0.5 mole) in isopropyl alcohol (700 ml) was made by stirring the fatty acid mixture in isopropyl alcohol. An aqueous solution of lysine in water was made by dissolving 73 grams of lysine (0.5 mole) in 200 ml of water. Both clear solutions were mixed with vigorous stirring resulting in the formation of thick white solids. The mixture was stirred for 12 hrs. The solvent was removed under reduced pressure and the resulting solid was crushed to fine powder. Yield 204 grams. Label the sample as Benemilk 417A.

### ii. PALMAC 640 /L-Lysine composition (molar ratio=2:1)

A solution of palmitic acid (PALMAC 640) 275.45 grams (1 mole) in isopropyl alcohol (1200 ml) was made by stirring palmitic acid in isopropyl alcohol. An aqueous solution of lysine in water was made by dissolving 73 grams of lysine (0.5 mole) in 200 ml of water. Both clear solutions were mixed with vigorous stirring resulting in the formation of thick white solids. The mixture was stirred for 12 hrs. The solvent was removed under reduced pressure and the resulting solid was crushed to fine powder. Yield 342 grams. Label the sample as Benemilk 417B.

### EXAMPLE 6: RUMEN BYPASS STUDY OF THE AMINO ACID COMPOSITIONS.

The samples were analyzed using a modification of the three-step in vitro procedure described by Miyazawa et al. (P# M254, A#1540). Samples were incubated in an Ankom Daisey incubator utilizing the XT4 bags. Nitrogen solubility was determined at each of the three phases in order to test replication and repeatability of the procedure. Residue remaining after 20 hours (ruminal phase), 22 hr (ruminal and abomasal phase) and 30 hr (ruminal, abomasal and intestinal phase) were freeze dried and analyzed for total nitrogen remaining. Dry matter content and total nitrogen of the samples is shown in Table 1. Nitrogen disappearance at the end of each of the three incubation phases can be found in Table 2.

**Table 1. Sample analysis**

| Sample | %Dry matter | % Nitrogen (%DM basis) | Ruminal phase - Nitrogen solubility (%) |
|---|---|---|---|
| 150A | 99.08 | 5.24 | 22.24 |
| | | | |
| 150B | 97.62 | 3.43 | 17.48 |
| | | | |
| 417A | 97.42 | 5.18 | 38.90 |
| | | | |
| 417B | 96.25 | 3.44 | 16.61 |
| | | | |
| 758A | 98.98 | 3.39 | 65.13 |
| | | | |
| 758B | 98.67 | 2.05 | 65.34 |
| | | | |
| 338A | 97.62 | 3.27 | 35.28 |
| | | | |
| 338B | 97.01 | 2.01 | 58.02 |

**Table 2. Data Set: %Nitrogen Disappearance**

| Sample | Ruminal Phase | Abomasal Phase | Intestinal Phase |
|---|---|---|---|
| 150 A | 24.20 | 39.70 | 20.17 |
| | 40.69 | 37.17 | 55.68 |
| | 30.97 | 30.06 | 34.64 |
| | 28.11 | 25.31 | 58.92 |
| **Mean** | **30.99** | **33.06** | **42.35** |
| 150B | 19.89 | 11.95 | 61.63 |
| | 8.97 | 16.38 | 30.04 |
| | 14.75 | 15.76 | 31.38 |
| | 19.58 | 16.73 | 37.11 |
| **Mean** | **15.80** | **15.21** | **40.04** |
| 417A | 38.06 | 39.45 | 72.59 |
| | 42.17 | 39.32 | 72.30 |
| | 22.83 | 41.12 | 73.83 |
| | 37.38 | 35.46 | 74.07 |
| **Mean** | **35.11** | **38.84** | **73.20** |
| 417B | 24.60 | 19.98 | 49.83 |
| | 24.55 | 23.64 | 45.75 |
| | 24.43 | 29.03 | 42.48 |
| | 23.87 | 27.96 | 53.35 |
| **Mean** | **24.36** | **25.15** | **47.85** |
| 758A | 73.76 | 85.16 | 91.20 |
| | 82.76 | 86.22 | 93.57 |
| | 91.44 | 82.28 | 96.34 |
| | 86.93 | 91.08 | 90.18 |
| **Mean** | **83.72** | **86.19** | **92.82** |
| 758B | 75.76 | 77.69 | 92.12 |
| | 84.28 | 76.37 | 90.38 |
| | 82.49 | 78.55 | 90.72 |
| | 79.11 | 82.15 | 93.59 |
| **Mean** | **80.41** | **78.69** | **91.70** |
| 338A | 71.61 | 92.39 | 80.64 |
| | 69.57 | 74.91 | 92.75 |
| | 64.76 | 74.06 | 82.44 |
| | 90.77 | 64.14 | 87.73 |
| **Mean** | **74.18** | **76.38** | **85.89** |
| 338B | 65.88 | 79.82 | 77.15 |
| | 81.11 | 65.79 | 90.31 |
| | 59.30 | 68.33 | 82.11 |
| | 79.35 | 65.99 | 88.71 |
| **Mean** | **71.41** | **69.98** | **84.57** |

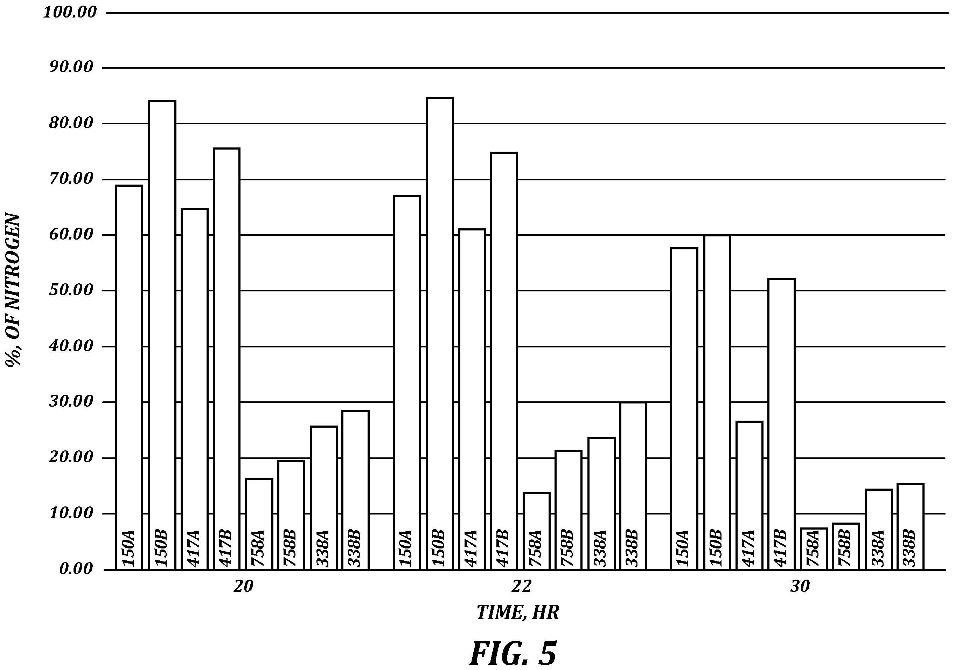

FIGURE 5 depicts the fraction of the original sample nitrogen remaining following the three incubation phases. Residue remaining after 20 hours (ruminal phase), 22 hr (ruminal and abomasal phase) and 30 hr (ruminal, abomasal and intestinal phase) are plotted.

## Claims

1. A rumen by-pass composition for a ruminant, comprising
an amine-organic acid salt including:
an amine compound, and
a fatty acid component, comprising an organic acid,
wherein the fatty acid component has a melting point not less than 40°C and an Iodine Value not greater than 45.

2. The rumen by-pass composition of Claim 1, wherein the fatty acid component comprises palmitic acid, stearic acid, oleic acid, linoleic acid, linolenic acid, eicosapentaenoic acid (EPA), docosahexaenoic acid (DHA), or a combination thereof.

3. The rumen by-pass composition of Claim 1, wherein the fatty acid component consists essentially palmitic acid, stearic acid, or a combination thereof.

4. The rumen by-pass composition of Claim 1, wherein the fatty acid component comprises from about 1% to about 50% by weight of an oleic acid component, wherein the oleic component comprises oleic acid, an oleic acid ester, a high oleic oil, or a combination thereof.

5. The rumen by-pass composition of Claim 1, wherein the fatty acid component comprises a high oleic oil, wherein the high oleic oil comprises at least 40% by weight of oleic content.

6. The rumen by-pass composition of Claim 1, wherein the organic acid comprises palmitic acid, stearic acid, oleic acid, linoleic acid, linolenic acid, eicosapentaenoic acid (EPA), or docosahexaenoic acid (DHA), or a combination thereof.

7. The rumen by-pass composition of Claim 1, wherein the amine compound comprises an amino acid, an amino acid derivative, or an amino acid precursor, wherein the amino acid comprises leucine, lysine, histidine, valine, arginine, threonine, isoleucine, phenylalanine, methionine, tryptophan, carnitine, alanine, asparagine, aspartic acid, cysteine, glutamic acid, glutamine, glycine, valine, ornithine, proline, selenocysteine, selenomethionine, serine, or tyrosine.

8. The rumen by-pass composition of Claim 1, wherein the amine compound comprises a lysine compound or a methionine compound, wherein the lysine compound comprises lysine, lysine ester, lysine amide, lysine imide, or a lysine precursor, and wherein the methionine compound comprises methionine, methionine ester, methionine amide, methionine imide, or a methionine precursor.

9. The rumen by-pass composition of Claim 1, wherein the amine-organic acid salt comprises a salt of a lysine compound and a long chain fatty acid having a carbon chain of at least 8 carbons.

10. The rumen by-pass composition of Claim 1, wherein the amine-organic acid salt comprises a salt of a methionine compound and a long chain fatty acid having a carbon chain of at least 8 carbons.

11. The rumen by-pass composition of Claim 1, comprising particles having an outer layer encapsulating at least one inner core, wherein the inner core comprises the amine-organic acid salt, wherein the outer layer comprises the fatty acid component, wherein the inner core comprises a higher level of the amine-organic acid salt than the outer layer.

12. The rumen by-pass composition of Claim 1, further comprising a surfactant component, a filler, an antistatic agent, a plasticizer, a colorant, an appetite stimulant, a flavoring agent, or a combination thereof, wherein the surfactant component comprises an emulsifier having a hydrophilic-lipophilic balance value of about 5 to about 25.

13. The rumen by-pass composition of Claim 12, wherein the surfactant component comprises polyoxyethylene stearate, polysorbate, polyoxyethylene sorbitan monolaurate, polyoxyethylene sorbitan monooleate, polyoxyethylene sorbitan monopalmitate, polyoxyethylene sorbitan monostearate, polyoxyethylene sorbitan tristearate, ammonium phosphatides, sodium or potassium or calcium salts of fatty acids, magnesium salts of fatty acids, mono- and diglycerides of fatty acids, acetic acid esters of mono- and diglycerides of fatty acids, lactic acid esters of mono- and diglycerides of fatty acids, citric acid esters of mono- and diglycerides of fatty acids, mono- and diacetyl tartaric acid esters of mono- and diglycerides of fatty acids, acetic acid esters of mono- and diglycerides of fatty acids, tartaric acid esters of mono- and diglycerides of fatty acids, sucrose esters of fatty acids sucroglycerides, polyglycerol esters of fatty acids polyglycerol polyricinoleate, propane-1,2-diol esters of fatty acids, thermally oxidised soya bean oil interacted with mono- and diglycerides of fatty acids, sodium stearoyl-2-lactylate, calcium stearoyl-2-lactylate, sorbitan monostearate, sorbitan tristearate, sorbitan monolaurate, sorbitan monooleate, sorbitan monopalmitate, or derivatives thereof.

14. The rumen by-pass composition of Claim 12, wherein the surfactant component comprises a surfactant derived from oleic acid.

15. The rumen by-pass composition of Claim 12, wherein the surfactant component comprises sodium oleate, potassium oleate, calcium oleate, ammonium oleate, sorbitan oleate, sorbitan trioleate, glyceryl oleate, methyl oleate, ethyl oleate, PEG oleate, triethanolamine oleate (TEA oleate), polysorbitan oleate, polysorbate 20, polysorbate 40, polysorbate 60, polysorbate 80, or a combination thereof.

## Patentansprüche

1. Pansen-Bypass-Zusammensetzung für einen Wiederkäuer, umfassend
- ein aminorganisches Säuresalz, beinhaltend:
- eine Aminverbindung und
- eine Fettsäurekomponente, die eine organische Säure umfasst,
wobei die Fettsäurekomponente einen Schmelzpunkt von nicht weniger als 40 °C und einen lodwert von nicht mehr als 45 hat.

2. Pansen-Bypass-Zusammensetzung nach Anspruch 1, wobei die Fettsäurekomponente Palmitinsäure, Stearinsäure, Oleinsäure, Linolsäure, Linolensäure, Eicosapentaensäure (EPA), Docosahexaensäure (DHA) oder eine Kombination davon umfasst.

3. Pansen-Bypass-Zusammensetzung nach Anspruch 1, wobei die Fettsäurekomponente im Wesentlichen aus Palmitinsäure, Stearinsäure oder einer Kombination davon besteht.

4. Pansen-Bypass-Zusammensetzung nach Anspruch 1, wobei die Fettsäurekomponente etwa 1 Gew.% bis etwa 50 Gew.-% einer Oleinsäurekomponente umfasst, wobei die Oleinkomponente Oleinsäure, einen Oleinsäureester, ein oleinreiches Öl oder eine Kombination davon umfasst.

5. Pansen-Bypass-Zusammensetzung nach Anspruch 1, wobei die Fettsäurekomponente ein oleinreiches Öl umfasst, wobei das ein oleinreiche Öl mindestens 40 Gew.-% Oleingehalt umfasst.

6. Pansen-Bypass-Zusammensetzung nach Anspruch 1, wobei die organische Säure Palmitinsäure, Stearinsäure, Oleinsäure, Linolsäure, Linolensäure, Eicosapentaensäure (EPA) oder Docosahexaensäure (DHA) oder eine Kombination davon umfasst.

7. Pansen-Bypass-Zusammensetzung nach Anspruch 1, wobei die Aminverbindung eine Aminosäure, ein Aminosäurederivat oder einen Aminosäurevorläufer umfasst, wobei die Aminosäure Leucin, Lysin, Histidin, Valin, Arginin, Threonin, Isoleucin, Phenylalanin, Methionin, Tryptophan, Carnitin, Alanin, Asparagin, Asparaginsäure, Cystein, Glutaminsäure, Glutamin, Glycin, Valin, Ornithin, Prolin, Selenocystein, Selenomethionin, Serin oder Tyrosin umfasst.

8. Pansen-Bypass-Zusammensetzung nach Anspruch 1, wobei die Aminverbindung eine Lysinverbindung oder eine Methioninverbindung umfasst, wobei die Lysinverbindung Lysin, Lysinester, Lysinamid, Lysinimid oder einen Lysinvorläufer umfasst und wobei die Methioninverbindung Methionin, Methioninester, Methioninamid, Methioninimid oder einen Methioninvorläufer umfasst.

9. Pansen-Bypass-Zusammensetzung nach Anspruch 1, wobei das aminorganische Säuresalz ein Salz einer Lysinverbindung und einer langkettigen Fettsäure mit einer Kohlenstoffkette von mindestens 8 Kohlenstoffen umfasst.

10. Pansen-Bypass-Zusammensetzung nach Anspruch 1, wobei das aminorganische Säuresalz ein Salz einer Methioninverbindung und einer langkettigen Fettsäure mit einer Kohlenstoffkette von mindestens 8 Kohlenstoffen umfasst.

11. Pansen-Bypass-Zusammensetzung nach Anspruch 1, umfassend Partikel mit einer Außenschicht, die mindestens einen Innenkern einkapselt, wobei der Innenkern das aminorganische Säuresalz umfasst, wobei die Außenschicht die Fettsäurekomponente umfasst, wobei der Innenkern einen höheren Wert des amin-organischen Säuresalzes als die Außenschicht umfasst.

12. Pansen-Bypass-Zusammensetzung nach Anspruch 1, weiter umfassend eine oberflächenaktive Komponente, einen Füllstoff, ein antistatisches Mittel, einen Weichmacher, ein Färbungsmittel, einen Appetitanreger, einen Aromastoff oder eine Kombination davon, wobei die oberflächenaktive Komponente einen Emulgator mit einem hydrophilen-lipophilen Gleichgewichtswert von etwa 5 bis etwa 25 aufweist.

13. Pansen-Bypass-Zusammensetzung nach Anspruch 12, wobei die oberflächenaktive Komponente Polyoxyethylenstearat, Polysorbat, Polyoxyethylensorbitanmonolaurat, Polyoxyethylensorbitanmonooleat, Polyoxyethylensorbitanmonopalmitat, Polyoxyethylensorbitanmonostearat, Polyoxyethylensorbitantristearat, Ammoniumphosphatide, Natrium- oder Kalium- oder Kalziumsalze von Fettsäuren, Magnesiumsalze von Fettsäuren, Mono- und Diglyceride von Fettsäuren, Essigsäureester von Mono- und Diglyceriden von Fettsäuren, Milchsäureester von Mono- und Diglyceriden von Fettsäuren, Zitronensäureester von Mono- und Diglyceriden von Fettsäuren, Mono- und Diacetylweinsäureester von Mono- und Diglyceriden von Fettsäuren, Essigsäureester von Mono- und Diglyceriden von Fettsäuren, Weinsäureester von Mono- und Diglyceriden von Fettsäuren, Sucroseester von Fettsäuresucroglyceriden, Polyglycerolester von Fettsäuren-Polyglycerolpolyricinoleat, Propan-1,2-Diolester von Fettsäuren, thermisch oxidiertes Sojabohnenöl, interagiert mit Mono- und Diglyceriden von Fettsäuren, Natriumstearoyl-2-lactylat, Kalziumstearoyl-2-lactylat, Sorbitanmonostearat, Sorbitantristearat, Sorbitanmonolaurat, Sorbitanmonooleat, Sorbitanmonopalmitat oder Derivate davon umfasst.

14. Pansen-Bypass-Zusammensetzung nach Anspruch 12, wobei die oberflächenaktive Komponente einen oberflächenaktiven Stoff umfasst, der von Oleinsäure abgeleitet ist.

15. Pansen-Bypass-Zusammensetzung nach Anspruch 12, wobei die oberflächenaktive Komponente Natriumoleat, Kaliumoleat, Kalziumoleat, Ammoniumoleat, Sorbitanoleat, Sorbitantrioleat, Glyceryloleat, Methyloleat, Ethyloleat, PEG-Oleat, Triethanolaminoleat (TEA-Oleat), Polysorbitanoleat, Polysorbat 20, Polysorbat 40, Polysorbat 60, Polysorbat 80 oder eine Kombination davon umfasst.

## Revendications

1. Composition pour ruminant destinée à court-circuiter le rumen, comprenant
un sel d'amine-acide organique incluant :
un composé d'amine, et
un composant à base d'acide gras, comprenant un acide organique,
dans laquelle le composant à base d'acide gras présente un point de fusion qui n'est pas inférieur à 40 °C et un indice d'iode qui n'est pas supérieur à 45.

2. Composition destinée à court-circuiter le rumen selon la revendication 1, dans laquelle le composant à base d'acide gras comprend de l'acide palmitique, de l'acide stéarique, de l'acide oléique, de l'acide linoléique, de l'acide linolénique, de l'acide eicosapentaénoïque (EPA), de l'acide docosahexaénoïque (DHA), ou une combinaison de ceux-ci.

3. Composition destinée à court-circuiter le rumen selon la revendication 1, dans laquelle le composant à base d'acide gras est constitué essentiellement par l'acide palmitique, l'acide stéarique, ou une combinaison de ceux-ci.

4. Combinaison destinée à court-circuiter le rumen selon la revendication 1, dans laquelle le composant à base d'acide gras comprend d'environ 1 % à environ 50 % en poids d'un composant à base d'acide oléique, dans laquelle le composant à base d'acide oléique comprend de l'acide oléique, un ester d'acide oléique, une huile oléique supérieure, ou une combinaison de ceux-ci.

5. Combinaison destinée à court-circuiter le rumen selon la revendication 1, dans laquelle le composant à base d'acide gras comprend une huile oléique supérieure, dans laquelle l'huile oléique supérieure comprend au moins 40 % en poids de teneur en acide oléique.

6. Composition destinée à court-circuiter le rumen selon la revendication 1, dans laquelle l'acide organique comprend de l'acide palmitique, de l'acide stéarique, de l'acide oléique, de l'acide linoléique, de l'acide linolénique, de l'acide eicosapentaénoïque (EPA), ou de l'acide docosahexaénoïque (DHA), ou une combinaison de ceux-ci.

7. Composition destinée à court-circuiter le rumen selon la revendication 1, dans laquelle le composé d'amine comprend un acide aminé, un dérivé d'acide aminé ou un précurseur d'acide aminé, dans laquelle l'acide aminé comprend de la leucine, de la lysine, de l'histidine, de la valine, de l'arginine, de la thréonine, de l'isoleucine, de la phénylalanine, de la méthionine, du tryptophane, de la carnitine, de l'alanine, de l'asparagine, de l'acide aspartique, de la cystéine, de l'acide glutamique, de la glutamine, de la glycine, de la valine, de l'ornithine, de la proline, de la sélénocystéine, de la sélénométhionine, de la sérine ou de la tyrosine.

8. Composition destinée à court-circuiter le rumen selon la revendication 1, dans laquelle le composé d'amine comprend un composé de lysine ou un composé de méthionine, dans laquelle le composé de lysine comprend de la lysine, un ester de lysine, un amide de lysine, un imide de lysine ou un précurseur de lysine, et dans laquelle le composé de méthionine comprend de la méthionine, un ester de méthionine, un amide de méthionine, un imide de méthionine, ou un précurseur de méthionine.

9. Composition destinée à court-circuiter le rumen selon la revendication 1, dans laquelle le sel d'amine-acide organique comprend un sel d'un composé de lysine et un acide gras à chaîne longue présentant une chaîne carbonée d'au moins 8 carbones.

10. Composition destinée à court-circuiter le rumen selon la revendication 1, dans laquelle le sel d'amine-acide organique comprend un sel d'un composé de méthionine et un acide gras à chaîne longue présentant une chaîne carbonée d'au moins 8 carbones.

11. Composition destinée à court-circuiter le rumen selon la revendication 1, comprenant des particules présentant une couche externe encapsulant au moins un noyau interne, dans laquelle le noyau interne comprend le sel d'amine-acide inorganique, dans laquelle la couche externe comprend le composant à base d'acide gras, dans laquelle le noyau interne comprend un niveau du sel d'amine-acide organique supérieur à la couche externe.

12. Composition destinée à court-circuiter le rumen selon la revendication 1, comprenant en outre un composant à base de tensioactif, une charge, un agent antistatique, un plastifiant, un colorant, une substance stimulant l'appétit, un aromatisant, ou une combinaison de ceux-ci, dans laquelle le composant à base de tensioactif comprend un émulsifiant présentant un indice d'équilibre hydrophile-lipophile d'environ 5 à environ 25.

13. Composition destinée à court-circuiter le rumen selon la revendication 12, dans laquelle le composant à base de tensioactif comprend du stéarate de polyoxyéthylène, du polysorbate, du monolaurate de polyoxyéthylène sorbitane, du monooléate de polyoxyéthylène sorbitane, du monopalmitate de polyoxyéthylène sorbitane, du monostéarate de polyoxyéthylène sorbitane, du tristéarate de polyoxyéthylène sorbitane, des phospholipides d'ammonium, des sels sodiques ou potassiques ou calciques d'acides gras, des sels magnésique d'acides gras, des mono- et diglycérides d'acides gras, des esters d'acide acétique de mono- et diglycérides d'acides gras, des esters d'acide lactique de mono- et diglycérides d'acides gras, les esters d'acide citrique de mono- et diglycérides d'acides gras, des esters d'acide mono- et diacétyltartrique de mono- et diglycérides d'acides gras, des esters d'acide acétique de mono- et diglycérides d'acides gras, des esters d'acide tartrique de mono- et diglycérides d'acides gras, des esters de sucrose d'acides gras, des sucroglycérides, des esters de polyglycérol d'acides gras, du polyricinoléate de polyglycérol, des esters de propane-1,2-diol d'acides gras, de l'huile de soja oxydée thermiquement ayant interagi avec des mono- et diglycérides d'acides gras, du stéaroyl-2-lactate de sodium, du stéaroyl-2-lactate de calcium, du monostéarate de sorbitane, du tristéarate de sorbitane, du monolaurate de sorbitane, du monooléate de sorbitane, du monopalmitate de sorbitane, ou des dérivés de ceux-ci.

14. Composition destinée à court-circuiter le rumen selon la revendication 12, dans laquelle le composant à base de tensioactif comprend un tensioactif dérivé de l'acide oléique.

15. Composition destinée à court-circuiter le rumen selon la revendication 12, dans laquelle le composant à base de tensioactif comprend de l'oléate de sodium, de l'oléate de potassium, de l'oléate de calcium, de l'oléate d'ammonium, de l'oléate de sorbitane, du trioléate de sorbitane, de l'oléate de glycéryle, de l'oléate de méthyle, de l'oléate d'éthyle, de l'oléate de PEG, de l'oléate de triéthanolamine (oléate de TEA), de l'oléate de polysorbitane, du polysorbate 20, du polysorbate 40, du polysorbate 60, du polysorbate 80 ou une combinaison de ceux-ci.
